# EUROPEAN PATENT APPLICATION

(11) **EP 4 356 918 A1**
(43) Date of publication of application: **24.04.2024**
(21) Application number: 22825354.8
(22) Date of filing: 16.06.2022
(51) Int. Cl.: A61K 35/747, A61K 35/17, A61K 36/068, A61K 36/8945, A61P 3/04, A23L 33/135, A23L 33/10, A23L 33/105

(54) **USE OF LACTOBACILLUS FERMENTUM STRAIN AND REGULATORY T CELLS IN COMBINATION THERAPY FOR PREVENTING AND TREATING METABOLIC DISEASE**

(30) Priority: 16.06.2021 KR 20210077914
(71) Applicant: GI Cell, Inc., Seongnam-si, Gyeonggi-do 13201 (KR); GI BIOME, Gyeonggi-do 13201 (KR)
(72) Inventor: JANG, Myung Ho, Seoul 05849 (KR); YANG, Bo-Gie, Seoul 05849 (KR); HONG, Chun-Pyo, Seongnam-si Gyeonggi-do 13643 (KR); KIM, Chea Ha, Seoul 08773 (KR); LEE, Dajeong, Seoul 04912 (KR); KIM, Hye Ri, Seoul 07275 (KR)
(74) Representative: Mewburn Ellis LLP
(86) International application number: PCT/KR2022/008555
(87) International publication number: WO 2022/265431

(57) **Abstract**

Disclosed is a composition containing a *Lactobacillus fermentum* strain, preferably a composition containing *Lactobacillus fermentum* GB-102 strain deposited with accession number KCTC 14105BP and regulatory T cells, and the use of co-administration of the strain and regulatory T cells for the suppression of weight gain or the prevention and/or treatment of metabolic diseases. The composition and the combination therapy have an effect of remarkably suppressing weight gain. Therefore, the composition and the combination therapy can be easily used to control the body weight of a subject, and are useful in the fields of beauty, livestock, medicines, and the like, in particular, are useful in the fields of pharmaceutical compositions, food additives, health functional foods, and therapeutic agents for ameliorating, preventing and treating metabolic diseases.

## Description

### BACKGROUND OF THE INVENTION

### Field of the Invention

The present invention relates to the use of a *Lactobacillus fermentum* strain for administration in combination with a regulatory T cells for the suppression of weight gain, and the prevention and/or treatment of metabolic diseases, and more preferably, to the use of a *Lactobacillus fermentum* GB-102 strain deposited with the accession number of KCTC 14105BP, and/or a *Lactobacillus fermentum* GB-103 strain deposited with the accession number of KCTC 14106BP and regulatory T cells for combination therapy and co-administration for the suppression of weight gain and the prevention or treatment of metabolic diseases.

### Description of the Related Art

Metabolic syndrome is a generic term for a cluster of risk factors specific to cardiovascular diseases caused by abnormalities or disorders of metabolism and it is reported that it is highly likely to lead to metabolic diseases such as cardiovascular disease, diabetes, and stroke. The National Heart, Lung and Blood Institute (NHLBI) and the American Heart Association (AHA) showed that metabolic syndrome is associated with obesity, impaired fasting blood sugar, high triglycerides, low HDL cholesterol, high fasting blood sugar, increased blood clotting, insulin resistance and the like.

Obesity is the most representative characteristic of patients with metabolic syndrome, and the main metabolic abnormality in obese patients is insulin resistance. When fat, especially abdominal visceral fat, accumulates excessively in the body, a large amount of free fatty acid flows into the hepatic portal blood. When blood fatty acids increase, the liver or muscle accepts fatty acids instead of glucose through insulin, which impedes influx of glucose, resulting in insulin resistance. Visceral adipocytes respond very sensitively to lipolysis stimulation, do not store triglycerides well and easily release free fatty acids. As a result, free fatty acids flow directly into the hepatic portal vein and interfere with the insulin signaling system, thus deteriorating the action of insulin. In addition, free fatty acids cause chronic inflammation in adipose tissue and produce various adipokines and cytokines that interfere with the insulin signaling system, thereby increasing insulin resistance and contributing to the induction of metabolic syndrome. In obese people, when insulin resistance occurs and glucose cannot enter the cells, blood glucose rises, causing diabetes. In addition, when insulin secretion increases to compensate for insulin resistance to increase the blood insulin concentration, the excretion of salt from the kidneys is inhibited and, as such, salt and water accumulate in the body. The sympathetic nerve is stimulated, causing constriction of blood vessels, high blood pressure, an increase in blood triglycerides and a decrease in HDL cholesterol. As a result, dyslipidemia occurs. Diabetes mellitus, hypertension, and dyslipidemia caused by insulin resistance act in combination to induce atherosclerosis, which leads to metabolic diseases such as angina and myocardial infarction in coronary arteries and cerebral infarction in cerebral arteries (J Clin Invest 2000; 106:171-76; Diabetes 1988;37:1595-607).

Therefore, the treatment of obesity is recognized as the most important target for the prevention and treatment of metabolic syndrome and related metabolic diseases. Current anti-obesity prescription drugs include Xenical^{®} (Roche Pharmaceuticals, Switzerland), Reductil^{®} (Abbott Laboratories Ltd, USA), and Exolise^{®} (Atopharma, France) and the like. The anti-obesity drugs are classified into appetite suppressant, energy consumption promoters, fat absorption inhibitors or the like, and most anti-obesity drugs are appetite suppressants that suppress appetite by regulating neurotransmitters related to the hypothalamus. However, conventional anti-obesity drugs have problems of causing side effects such as heart disease, respiratory disease, and nervous system disease, and having low durability. Accordingly, there is a need for the development of safe and effective anti-obesity drugs.

Meanwhile, research has been actively conducted on probiotics to prevent or treat obesity and metabolic diseases using bacteria (e.g., lactic acid bacteria) that have been considered safe microorganisms. In particular, research results have reported that lactic acid bacteria have effects such as maintaining normal intestinal flora, improving intestinal flora, exhibiting anti-diabetic and anti-lipidemic activities, inhibiting carcinogenesis and colon cancer, exhibiting non-specific activity of the host's immune system and the like.

The WHO defines live microorganisms that are beneficial to the health of the host when administered in appropriate amounts as "probiotics" (Nat Rev Gastroenterol Hepatol 11, 506-514 (2014)). Lactobacillus species are lactic acid bacteria (LAB) belonging to the family Lactobacillaceae. Lactobacillus is a well-known microbial family with a distinct ecological status. Several lactic acid bacteria are known to be traditionally associated with foods such as milk, dairy products, fermented foods, and sausages. Lactobacilli are a group of microorganisms generally regarded as safe (GRAS) by the FDA and are widely used in food and other industries. Lactobacilli are classified as facultative anaerobe, non-spore-forming, non-motile, rod-shaped, gram-positive bacteria, and are generally catalase-negative. Lactobacillus may be homofermentative or heterofermentative, and produce lactic acid as a final product of primary fermentation (Front Cell Infect Microbiol 2, 86 (2012)). Lactobacillus exhibits smooth and convex colonies.

Regarding the *Lactobacillus* strains associated with the prophylactic or therapeutic effects of obesity or metabolic diseases, Korean Patent No. 10-1494279 discloses a *Lactobacillus plantarum* KY1032 strain (Accession No.: KCCM-10430) having the effect of inhibiting adipocyte differentiation, Korean Patent No. 10-0996577 discloses *Lactobacillus kerbetus* HY7601 (Accession No.: KCTC11456BP) having an anti-obesity effect, and Korean Patent No. 10-1394348 discloses a *Lactobacillus plantarum* DSR920 strain (Accession No.: KCCM 11210P) having an effect of inhibiting adipocyte differentiation. It is reported that each strain has anti-obesity activity through an independent mechanism for each strain, but the effect has not reached a sufficient level to be commercially successful.

Against this background, the present inventors identified novel *Lactobacillus fermentum* strains (Accession Nos. KCTC 14105BP and KCTC 14106BP), found that the strains have prophylactic or therapeutic effects for obesity and metabolic diseases and filed a patent application (Korean Patent Application Nos. 10-2021-0086537 and 10-2021-0086539) based thereon. As a result of extensive efforts to improve the effects of suppressing weight gain and preventing and treating metabolic diseases by the *Lactobacillus fermentum* (*Limosilactobacillus fermentum*) strain, the present inventors found that, when a combination of the strain with regulatory T cells (Treg cells) is administered, the ability to control blood sugar level (glucose tolerance) and the ability to suppress weight gain are greatly improved after glucose administration, compared to when the strain or regulatory T cells are administered alone, and that prevention and treatment of metabolic diseases including obesity and insulin resistance are possible based thereon. The present invention was completed based on this finding.

The information disclosed in this Background section is provided only for better understanding of the background of the present invention, and therefore it may not include information that forms the prior art that is already obvious to those skilled in the art.

### SUMMARY OF THE INVENTION

Therefore, the present invention has been made in view of the above problems, and it is one object of the present invention to provide the use of a *Lactobacillus fermentum* strain for administration in combination with a regulatory T cells for the suppression of weight gain.

It is another object of the present invention to provide a composition for suppressing weight gain containing a *Lactobacillus fermentum* strain and regulatory T cells.

It is another object of the present invention to provide the use of a *Lactobacillus fermentum* strain and regulatory T cells for the preparation of a composition for suppressing weight gain.

It is another object of the present invention to provide a method of suppressing weight gain using a *Lactobacillus fermentum* strain and regulatory T cells.

It is another object of the present invention to provide the use of a *Lactobacillus fermentum* strain for administration in combination with regulatory T cells for the prevention or treatment of metabolic diseases.

It is another object of the present invention to provide a composition for preventing or treating metabolic diseases containing a *Lactobacillus fermentum* strain and regulatory T cells.

It is another object of the present invention to provide the use of a *Lactobacillus fermentum* strain for the preparation of the composition for preventing or treating metabolic diseases.

It is another object of the present invention to provide a method for preventing or treating metabolic diseases using a *Lactobacillus fermentum* strain and regulatory T cells.

In order to achieve the above object, the present invention provides a composition for suppressing weight gain containing a *Lactobacillus fermentum* strain as an active ingredient, wherein the composition is administered in combination with regulatory T cells.

The present invention also provides a composition for suppressing weight gain containing a *Lactobacillus fermentum* strain and regulatory T cells as active ingredients.

The present invention also provides the use of a *Lactobacillus fermentum* strain for administration in combination with regulatory T cells for the suppression of weight gain and the use of a *Lactobacillus fermentum* strain for the preparation of the composition for suppressing weight gain.

The present invention also provides a method of suppressing weight gain including administering a *Lactobacillus fermentum* strain in combination with regulatory T cells to a subject.

The present invention also provides a composition for ameliorating, preventing or treating metabolic diseases containing a *Lactobacillus fermentum* strain as an active ingredient, the composition is administrated in combination with regulatory T cells.

The present invention also provides a composition for ameliorating, preventing or treating metabolic diseases containing a *Lactobacillus fermentum* strain and regulatory T cells as active ingredients.

The present invention also provides the use of a *Lactobacillus fermentum* strain for administration in combination with regulatory T cells for the amelioration, prevention or treatment of metabolic diseases.

The present invention also provides a method of ameliorating, preventing or treating metabolic diseases including administering a *Lactobacillus fermentum* strain in combination with regulatory T cells to a subject.

### BRIEF DESCRIPTION OF THE DRAWINGS

The above and other objects, features and other advantages of the present invention will be more clearly understood from the following detailed description taken in conjunction with the accompanying drawings, in which:

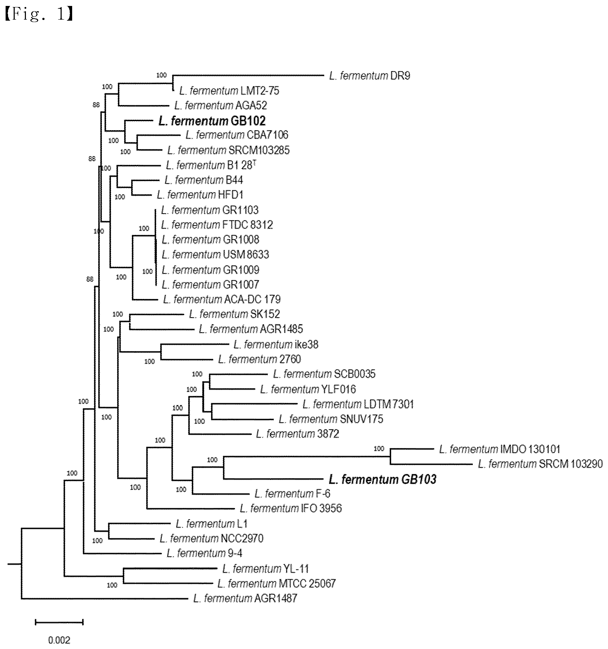

FIG. 1 is a phylogenetic tree of *Lactobacillus fermentum* GB-102 and GB-103 strains according to the present invention and a known conventional *Lactobacillus sp.* strain;

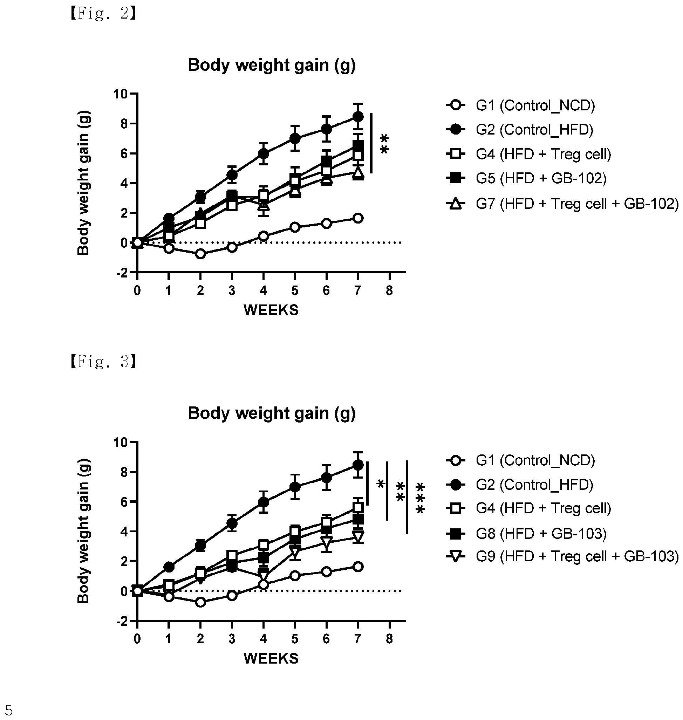

FIG. 2 is a graph showing a change in body weight of mice with obesity induced through free feeding of a high-fat diet for 8 weeks when administering the GB-102 strain and Treg cells alone or in combination thereto. Statistical analysis of weight change was performed using ANOVA. Groups were compared with one another using a Student t-test. The resulting value is expressed as mean ± SEM (where n > 5 in each group). It is considered that there is a significant difference when a P value is less than 0.05;
FIG. 3 shows the blood glucose control effect confirmed through glucose tolerance test by measuring blood glucose at the tail end of mice, to which glucose is administered intraperitoneally, when the GB-103 strain and Treg cells are administered to the mice alone or in combination. Statistical analysis was performed using ANOVA. Groups were compared with one another using Student's t-test. The resulting value is expressed as mean ± SEM (where n > 5 in each group). It is considered that there is a significant difference when a P value is less than 0.05;

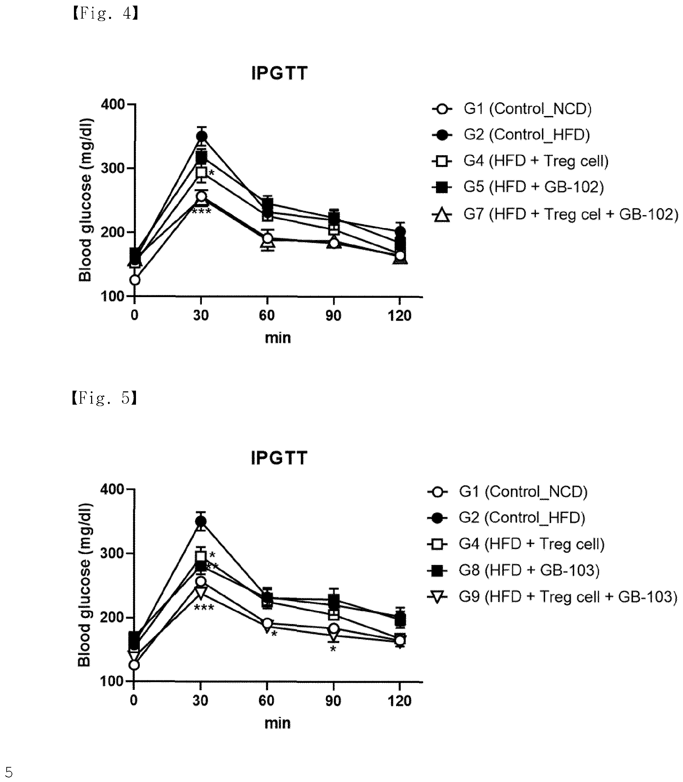

FIG. 4 shows the blood glucose control effect confirmed through glucose tolerance test by measuring blood glucose at the tail end of mice, to which glucose is administered intraperitoneally, when GB-102 and Treg cells are administered to the mice alone or in combination. Statistical analysis was performed using one-way ANOVA. When the results were considered significant, the respective groups were compared with one another using Dunnett's multiple comparison test and a post-hoc test was performed. The resulting value is expressed as mean ± SEM (where n > 5 in each group). It is considered that there is a significant difference when a P value is less than 0.05; and
FIG. 5 shows the blood glucose control effect confirmed through glucose tolerance test by measuring blood glucose at the tail end of mice, to which glucose is administered intraperitoneally, when the GB-103 strain and Treg cells are administered to the mice alone or in combination. Statistical analysis was performed using one-way ANOVA. When the results were considered significant, the respective groups were compared with one another using Dunnett's multiple comparison test and a post-hoc test was performed. The resulting value is expressed as mean ± SEM (where n > 5 in each group). It is considered that there is a significant difference when a P value is less than 0.05 (**p* < 0.05; ***p* < 0.01; ****p* < 0.001).

### DETAILED DESCRIPTION OF THE INVENTION

Unless defined otherwise, all technical and scientific terms used herein have the same meanings as appreciated by those skilled in the field to which the present invention pertains. In general, the nomenclature used herein is well-known in the art and is ordinarily used.

In concentration ranges described herein, the term "to" is intended to include critical ranges of both a lower limit and an upper limit (not less than the lower limit and not more than the upper limit). Unless the both critical ranges are included, the concentration range is described as "higher than" and "lower than". As used herein, the term "about" for a numerical value is used to include a range expected by those skilled in the art to have an effect substantially equivalent to a given numerical value, for example, ±20%, ±10%, ±5%, or the like of the given numerical value, but is not limited thereto.

Methods for ameliorating, preventing and treating metabolic diseases such as obesity using microorganisms are attracting a great deal of attention as substitutes for conventional surgical therapy or compound-based drug therapy. However, microorganisms can exhibit very diverse activities and side effects depending on the species thereof, and can exhibit very diverse activities even in the same species and various side effects depending on the condition of the subject to whom they are administered (Tze Guan Tan, 113(50) :E8141-E8150, 2016). For this reason, it is necessary to clearly identify a specific strain and the prophylactic and therapeutic effects thereof for metabolic diseases.

Recently, for example, strains exhibiting effects such as inhibiting adipocyte differentiation, inhibiting lipase enzymes and having lipolysis activity have been reported, but administration of the strain alone is insufficient to obtain the effect of preventing or treating metabolic diseases.

In Example of the present invention, the present inventors found that the effects of greatly suppressing weight gain and of controlling blood glucose (glucose tolerance) were observed in a high-fat-diet animal model administered a combination of regulatory T cells (Treg cells) and a *Lactobacillus fermentum* strain.

Obesity is known to cause excessive fat accumulation in skeletal muscle and liver, and inhibit the insulin signaling pathway according to the increase of lipid metabolites such as free fatty acid (acyl-CoA), DAG (diacylglycerol), and ceramide during fat accumulation metabolism, thus inducing the development of insulin resistance (Proc. Nutr. Soc. 63: 375-380; J. Lipid Res. 50:S74-S79; Diabetes 60: 2588-2597).

Accordingly, in one aspect, the present invention is related to a composition for suppressing weight gain containing a *Lactobacillus fermentum* strain as an active ingredient, wherein the composition is administered in combination with regulatory T cells. The present invention is characterized in that, when the *Lactobacillus fermentum* strain is administered in combination with regulatory T cells, the inhibitory activity against weight gain is greatly improved.

As used herein, the term "*Lactobacillus fermentum*" may be used interchangeably with "*Limosilactobacillus fermentum*" since it has the same meaning.

In the present invention, the *Lactobacillus fermentum* strain preferably includes at least one selected from the group consisting of a *Lactobacillus fermentum* GB-102 strain deposited with the accession number of KCTC 14105BP and a *Lactobacillus fermentum* GB-103 strain deposited with the accession number of KCTC 14106BP.

As used herein, the term "regulatory T cells (Treg cells)" refers to a type of differentiated T cells, which serves to suppress the immune function, to maintain tolerance to autoantigens and to inhibit the onset of autoimmune diseases. Regulatory T cells generally express immunosuppressive cytokines such as IL-10, and suppress the induction and proliferation of effector T cells. In the present invention, the regulatory T cells may be naturally derived from animals, but are not limited thereto, and include recombinant regulatory T cells, for example, CAR Treg cells, prepared through cell engineering, gene engineering and the like.

As used herein, the term "administration in combination with", "combination administration" or "co-administration" means that two or more types of active ingredients are administered simultaneously or sequentially, or administered at a specific interval to obtain an improved effect when each active ingredient is independently administered, based on the action of two or more types of active ingredients.

In the present invention, the co-administration may be performed by administration of the *Lactobacillus fermentum* strain in combination with regulatory T cells, and may be further used in combination with a composition containing other active ingredients or other therapies.

In the present invention, the *Lactobacillus fermentum* strain, which is administered in combination with regulatory T cells may be prepared to be incorporated into a single formulation with the regulatory T cells, or may be each prepared into a composition of an independent formulation and then administered in combination. For example, the composition containing the *Lactobacillus fermentum* strain may be prepared as a formulation for oral administration, and the regulatory T cells may be prepared as a formulation for intravenous administration, without being limited thereto.

In the present invention, the composition containing the *Lactobacillus fermentum* strain is preferably prepared in an oral formulation, but is not limited thereto. In the present invention, the composition containing the *Lactobacillus fermentum* strain may be used in the form of a composition suitable for administration to humans or animals.

The *Lactobacillus fermentum* strain, which is an active ingredient of the composition of the present invention, may be provided in the form of a lyophilizate, capsule, a cultured suspension, a fermented solution, a dry powder or a granule as an oral formulation.

When a parenteral formulation of the *Lactobacillus fermentum* strain, which is the active ingredient of the composition of the present invention, is prepared, it may contain a sterile aqueous solution, a non-aqueous solvent, a suspension, an emulsion, a lyophilizate, and a suppository. Examples of the non-aqueous solvent and suspension include propylene glycol, polyethylene glycol, vegetable oil such as olive oil, injectable esters such as ethyl oleate, and the like. Examples of the suppository base include Witepsol, macrogol, Tween 61, cacao butter, laurin butter, glycerogelatin and the like. Suitable formulations known in the art may be prepared according to the method disclosed in the document (Remington's Pharmaceutical Science (latest edition), Mack Publishing Company, Easton PA).

In the present invention, the composition may further contain suitable carriers, excipients and diluents commonly used in addition to the *Lactobacillus fermentum* strain.

In the present invention, the regulatory T cells may be prepared as a separate formulation for administration in combination with the composition of the present invention. In the present invention, the regulatory T cells may be prepared as an injection formulation containing the same, preferably a formulation for intravenous administration, but is not limited thereto. The separate composition containing the regulatory T cells may further contain suitable commonly used carriers, excipients and diluents that can be administered to humans or animals, and may further contain an additional ingredient capable of maintaining the survival or activity of regulatory T cells.

Examples of the carrier, excipient or diluent according to the present invention include lactose, dextrose, sucrose, sorbitol, mannitol, xylitol, erythritol, maltitol, starch, acacia rubber, alginate, gelatin, calcium phosphate, calcium silicate, cellulose, methyl cellulose, microcrystalline cellulose, polyvinyl pyrrolidone, water, methylhydroxybenzoate, propylhydroxybenzoate, talc, magnesium stearate, mineral oil, and the like. The formulation of the composition may be prepared using a commonly used diluent or excipient such as a filler, extender, binder, wetting agent, disintegrant, or surfactant. In the present invention, the composition may further contain a lubricant, a wetting agent, an emulsifier, a suspending agent, a preservative, a sweetening agent or a flavoring agent. The pharmaceutical composition of the present invention may be prepared as an enteric coating formulation using a method known in the art so that the microorganism, which is an active ingredient, can be rapidly released into the intestines after passing through the stomach and reaching the small intestine.

In addition, the composition of the present invention may be prepared as a capsule using a conventional encapsulation method. For example, pellets containing the freeze-dried microorganisms of the present invention are prepared using standard carriers and then charged in hard gelatin capsules. Alternatively, a suspension or dispersion may be prepared using the microorganism of the present invention along with any suitable pharmaceutical carrier, such as aqueous gum, cellulose, silicate or oil, and then may be charged into soft gelatin capsules.

In the present invention, the composition may be provided as an enteric coated preparation, in particular, as a unit oral formulation. As used herein, the term "enteric coating" includes all types of pharmaceutically acceptable coatings that are not degraded by gastric acid, but are sufficiently degraded in the small intestine to release the active ingredient into the small intestine. The material for the enteric coating may be appropriately selected from known polymer materials and suitable polymer materials are described in a number of known publications (L. Lachman et al., The Theory and Practice of Industrial Pharmacy, 3rd ed., 1986, pp. 365-373; H. Sucker et al., Pharmazeutische Technologie, Thieme, 1991, pp. 355-359; Hagers Handbuch der pharmazeutischen Praxis, 4th ed., Vol. 7, pp. 739-742, and 766-778, (Springer Verlag, 1971); and Remington's Pharmaceutical Sciences, 13th ed., pp. 1689-1691 (Mack Publ., Co., 1970)), and the suitable polymer materials may comprise cellulose ester derivatives, cellulose ethers, methyl acrylate copolymers of acrylic resins, and copolymers of maleic acid and phthalic acid derivatives but are not limited thereto.

In the present invention, the *Lactobacillus fermentum* strain and regulatory T cells are administered through an independent route. Each active ingredient may be independently administered in an appropriate dosage using a suitable administration regimen by those skilled in the art. For example, as in one embodiment of the present invention, preferably, the *Lactobacillus fermentum* strain is administered orally, and the regulatory T cells are administered through intravenous injection, but the present invention is not limited thereto.

In the present invention, the co-administration may be performed by simultaneously administering the *Lactobacillus fermentum* strain and regulatory T cells.

In the present invention, the co-administration may be performed by sequentially administering the *Lactobacillus fermentum* strain and regulatory T cells. For example, regulatory T cells are administered after administration of the *Lactobacillus fermentum* strain, or the *Lactobacillus fermentum* strain is administered after the administration of regulatory T cells. In the present invention, when the *Lactobacillus fermentum* strain and the regulatory T cells are sequentially administered, they are administered at a time interval, which includes, but is not limited to, a 1-minute interval, a 5-minute interval, a 10-minute interval, a 20-minute interval, a 30-minute interval, a 1-hour interval, a 1-day interval, a several-day interval, or a several-week interval, and are administered sequentially at appropriate intervals by those of ordinary skill in the art.

In the present invention, the administration may be repeatedly performed one or more times. When the active ingredients of the present invention, the *Lactobacillus fermentum* strain and regulatory T cells, are repeatedly administered, the administration interval can be easily adjusted by those skilled in the art according to the condition of the subject to which they are administered and the levels of the desired effect. For example, the administration interval may be a 1-hour interval, a 6-hour interval, an 8-hour interval, a 12-hour interval, a 1-day interval, a 2-day interval, a 1-week interval, a 2-week interval, or a 1-month interval, but is not limited thereto. In the present invention, the *Lactobacillus fermentum* strain and the regulatory T cells may be each administered at an independent administration interval. In an embodiment of the present invention, the *Lactobacillus fermentum* strain is administered once a day, and the regulatory T cells are administered once a week, but the present invention is not limited thereto.

In the present invention, the *Lactobacillus fermentum* strain and regulatory T cells are each administered in an effective dose. In the present invention, the term "effective dose" may mean a dose not less than the minimum dose which is required to obtain the desired effect of the present invention, that is, the effect of inhibiting weight gain, or the effect of preventing or treating metabolic diseases. In the present invention, when the composition is a food composition or a pharmaceutical composition, it is administered in a cytologically effective dose or a pharmaceutically effective dose, respectively.

In the present invention, the composition containing the *Lactobacillus fermentum* strain may be administered in the form of a composition, for example, a pharmaceutical composition or a food composition, independent of a formulation of Treg cells administered in combination therewith. Preferably, the pharmaceutical composition containing the *Lactobacillus fermentum* strain and the pharmaceutical composition containing Treg cells are each administered at an effective dose, or a food composition containing the *Lactobacillus fermentum* strain and a pharmaceutical composition containing Treg cells are administered at an effective dose, but the present invention is not limited thereto.

For example, in the present invention, the *Lactobacillus fermentum* strain is administered at a dose of about 1×10¹ CFU to about 1×10²⁰ CFU, preferably about 1×10⁴ CFU to about 1×10¹⁶ CFU, more preferably about 1×10⁶ CFU to about 1× 10¹² CFU. In one embodiment of the present invention, the *Lactobacillus fermentum* strain is administered in a dose of about 5×10⁹ CFU/head/200 µl, but is not limited thereto.

In the present invention, the *Lactobacillus fermentum* strain may be contained in an amount of 10⁴ to 10¹⁶ CFU/g, preferably 10⁶ to 10¹² CFU/g with respect to the total amount of the composition, or a culture containing an equivalent number of viable bacteria may be contained in the composition. In general, 1×10⁶ CFU/g or more of viable bacteria, preferably 1×10⁸ to 1×10¹² CFU/g of viable bacteria, may be administered once or several times in divided portions to an adult patient.

For example, in the present invention, the regulatory T cells are administered in an amount of about 1×10¹ cells to about 1x 10⁵⁰ cells, preferably about 1×10³ cells to about 1x 10³⁰ cells, more preferably about 1×10⁴ cells to about 1x 10¹⁰ cells. In one embodiment of the present invention, the regulatory T cells are administered in an amount of about 1×10⁶ cell/head/200 µl, but the present invention is not limited thereto.

As used herein, the term "suppression of weight gain" refers to suppression of weight gain due to various causes such as excessive sugar intake, fat accumulation, and hormone changes. In the present invention, the suppression of weight gain may mean a significant decrease in weight gain compared to when the composition for suppressing weight gain of the present invention is not administered.

In addition, in the present invention, the suppression of weight gain is used to include a decrease in body weight, and thus the composition for suppressing weight gain of the present invention exhibits a weight loss effect.

In the present invention, the composition may be used as a pharmaceutical composition or a food composition for suppressing weight gain and/or facilitating weight loss, and the amount and form that is used therefor may be appropriately controlled depending on the purpose.

The composition according to the present invention is effective in suppressing weight gain and facilitating weight loss and can be administered for a long period of time since it has almost no toxicity and side effects due to drugs.

In the present invention, the composition may be used as a pharmaceutical composition or a food composition for suppressing weight gain and/or facilitating weight loss.

The pharmaceutical composition according to the present invention may be administered in a pharmaceutically effective amount, and the term "pharmaceutically effective amount" refers to an amount sufficient for treating a disease at a reasonable benefit/risk ratio applicable to all medical treatments, and the effective dosage level may be determined depending on a variety of factors including the type of the disease of the patient, the severity of the disease, the activity of the drug, the sensitivity of the patient to the drug, the administration time, the administration route, the excretion rate, the treatment period, drugs used concurrently therewith, and other factors well-known in the pharmaceutical field. The pharmaceutical composition of the present invention may be administered as a single therapeutic agent or in combination with other therapeutic agents, may be administered sequentially or simultaneously with a conventional therapeutic agent, and may be administered in single or multiple doses. Taking into consideration these factors, it is important to administer the minimum amount sufficient to achieve maximum efficacy without side effects and the amount can be easily determined by those skilled in the art.

As used herein, the term "food composition" is used in a broad sense to encompass substances containing nutrients, and examples of the food composition include meat, sausage, bread, chocolate, candy, snacks, confectionery, pizza, ramen, other noodles, gum, dairy products including ice cream, various soups, beverages, tea, drinks, alcoholic beverages, vitamin complexes, prebiotics, probiotics, postbiotics, health supplement food, health functional food, health food and the like. The term "food composition" is used in an ordinary sense to encompass "food" and "food additive" or "composition for food additive" added to food.

In the present invention, when the food is provided as feed for animals other than humans, it may be used interchangeably in substantially the same sense as "feed". Therefore, in the present invention, the food composition may mean a feed composition or feed additive (feed additive composition).

In the present invention, the food composition may be a health functional food having an activity of inhibiting weight gain or facilitating weight loss.

As used herein, the term "functional food" has the same meaning as the term "food for special health use (FoSHU)" and refers to a food having strong medical and pharmaceutical effects that has been processed to efficiently provide a bioregulatory function as well as a nutrition supply function. Here, the term "functional" means obtaining beneficial effects for health purposes, such as controlling nutrients or exhibiting physiological effects with regard to the structures and functions of the human body. The food of the present invention may be prepared by a method commonly used in the art, and the preparation may be performed using raw materials and ingredients commonly added in the art. In addition, the food may also be prepared into any formulation recognized as a food without limitation, and the health functional food according to the present invention may be in the form of a powder, granule, tablet, capsule, or beverage.

The term "health food" refers to a food having an active health maintenance or promotion effect beyond that of a general food, and the term "health supplement food" refers to a food ingested for the purpose of health supplement. In some cases, the terms "health functional food", "health food", and "health supplement food" are used interchangeably.

The food composition may further contain a physiologically acceptable carrier, and there is no particular limitation as to the kind of carrier, and any carrier commonly used in the art may be used.

In addition, the composition may contain additional ingredients that are commonly used in food compositions to improve smell, taste, visual quality (appearance), and the like. For example, the composition may contain vitamins A, C, D, E, B1, B2, B6 and B12, niacin, biotin, folate, pantothenic acid, and the like. In addition, the composition may contain minerals such as zinc (Zn), iron (Fe), calcium (Ca), chromium (Cr), magnesium (Mg), manganese (Mn), copper (Cu), and chromium (Cr). In addition, the composition may contain amino acids such as lysine, tryptophan, cysteine, and valine.

In addition, the composition may contain food additives such as preservatives (potassium sorbate, sodium benzoate, salicylic acid, sodium dehydroacetate, etc.), disinfectants (bleached powder and highly bleached powder, sodium hypochlorite, etc.), antioxidants (butylhydroxyanisole (BHA), butylhydroxytoluene (BHT), etc.), colorants (tar color, etc.), color-developing agents (sodium nitrite, sodium nitrite, etc.), bleaching agents (sodium sulfite), seasonings (MSG, etc.), sweeteners (dulcin, cyclamate, saccharin, sodium, etc.), flavorings (vanillin, lactones, etc.), expanding agents (alum, D-potassium hydrogen tartrate, etc.), reinforcing agents, emulsifiers, thickeners, coating agents, gum base agents, anti-foaming agents, solvents, and enhancers. The additive may be selected according to the type of food and used in an appropriate amount.

In addition to the polysaccharide of the present invention, the composition may further contain a cytologically acceptable food supplement additive, may be used in combination with other foods or food ingredients, and may be appropriately used according to a conventional method. The amount of the active ingredient that is mixed may be appropriately determined according to the purpose of use (prevention, health or therapeutic treatment).

In another aspect, the present invention is related to a composition for suppressing weight gain containing a *Lactobacillus fermentum* strain and regulatory T cells.

In another aspect, the present invention is related to the use of the *Lactobacillus fermentum* strain for administration in combination with regulatory T cells for the suppression of weight gain and the use of the *Lactobacillus fermentum* strain and regulatory T cells for the preparation of the composition for suppressing weight gain.

In another aspect, the present invention is related to a method of suppressing weight gain, the method including administering a *Lactobacillus fermentum* strain in combination with regulatory T cells to a subject.

In the present invention, the *Lactobacillus fermentum* strain preferably includes at least one selected from the group consisting of a *Lactobacillus fermentum* GB-102 strain deposited with the accession number of KCTC 14105BP and a *Lactobacillus fermentum* GB-103 strain deposited with the accession number of KCTC 14106BP.

The method for suppressing weight gain according to the present invention is intended to include a method for reducing weight. Therefore, the method of the present invention may be used to reduce the weight of a subject.

In the present invention, the administration may be performed through various routes. The mode of administration may be an oral or parenteral administration, and the parenteral route may be, for example, subcutaneous, intravenous, intramuscular, intrauterine intrathecal or intracerebrovascular injection, but is not limited thereto. In the present invention, the regimen, the dose, and the number of administration may be determined depending on various related factors such as the age, gender and weight of the patient and the severity of the disease.

In the present invention, the co-administration may be performed by administration of the *Lactobacillus fermentum* strain in combination with immune cells, and may be further used in combination with a composition containing other active ingredients or other therapies.

In the present invention, unless otherwise specified, the co-administration may have the same features as those described in terms of the composition for suppressing weight gain of the present invention.

In the present invention, the method for suppressing weight gain may be used in combination with various therapies for suppressing weight gain other than the method of the present invention or various known agents for suppressing weight gain.

The composition for suppressing weight gain and the method for suppressing weight gain according to the present invention exhibit a remarkable weight control effect and thus can be used for the prevention or treatment of obesity.

In another embodiment of the present invention, the result of intraperitoneal glucose tolerance test (IPGTT) showed that the co-administration of the *Lactobacillus fermentum* strain and regulatory T cells has effects of suppressing weight gain and controlling blood sugar.

The intraperitoneal glucose tolerance test is the most common method for confirming insulin resistance of a subject. Lipid metabolites produced during the metabolism of triglycerides (Proc. Nutr. Soc. 63: 375-380), specifically free fatty acids (acyl-CoA), diacylglycerol (DAG), ceramide, and the like are reported to inhibit the insulin signaling pathway to induce the development of insulin resistance (Diabetes 60: 2588-2597). Obesity has a cause or effect relationship with insulin resistance, and therefore, in general, obesity and insulin resistance are observed at the same time in most cases. Obesity and insulin resistance are major causes of various metabolic diseases such as diabetes, hypertension, arteriosclerosis, hyperlipidemia, hyperinsulinemia, insulin resistance, metabolic inflammation, impaired fasting blood glucose, impaired glucose tolerance and glucose tolerance syndrome.

As a result, the effects of suppressing weight gain (or weight loss) and improving blood sugar control ability through the co-administration of the *Lactobacillus fermentum* strain and regulatory T cells of the present invention indicate that the co-administration can be used for the prevention or treatment of metabolic diseases.

Therefore, in another aspect, the present invention is related to a composition for preventing or treating metabolic diseases containing a *Lactobacillus fermentum* strain, wherein the composition is administered in combination with regulatory T cells.

In the present invention, the *Lactobacillus fermentum* strain preferably includes at least one selected from the group consisting of a *Lactobacillus fermentum* GB-102 strain deposited with the accession number of KCTC 14105BP and a *Lactobacillus fermentum* GB-103 strain deposited with the accession number of KCTC 14106BP.

In the present invention, the metabolic disease refers to a disease caused by an abnormality in metabolism. In the present invention, the "metabolic disease" may be used interchangeably with "metabolic syndrome" since it has substantially the same meaning as the same. In the present invention, the metabolic disease is a disease presumed to be caused by insulin resistance and shows a symptom of abnormality in two or more of cholesterol, blood pressure, and blood sugar. The metabolic disease refers to a cluster of conditions that occur together, increasing risk factors of various cardiovascular diseases and type 2 diabetes, and is used to encompass insulin resistance and various complicated metabolic abnormalities and clinical aspects related thereto.

In the present invention, the metabolic disease may be, for example, a cardiovascular disease or a glucose metabolism abnormality, and more specifically, may be selected from the group consisting of obesity, diabetes, hypertension, arteriosclerosis, hyperlipidemia, hyperinsulinemia, insulin resistance, fasting blood glucose disorder, impaired glucose tolerance and glucose tolerance syndrome, but is not limited thereto, and includes diseases that result from weight gain or glucose control or have weight gain or glucose control as main symptoms.

As used herein, the term "cardiovascular disease (CVD)" refers to a circulatory system disease related to the heart and blood vessels and, for example, includes hypertension, ischemic heart disease, coronary artery disease, angina pectoris, myocardial infarction, atherosclerosis, arrhythmia, cerebrovascular disease, stroke, and the like, without being limited thereto.

As used herein, the term "obesity" is a disease characterized by excessive fat accumulation, and the WHO defines obesity as a body mass index (BMI) of 30 or more. Obesity is also defined based on the waist circumference (WC) or the like (Korean Society for Obesity) . Accordingly, obesity will be considered as having a broad meaning including all of the conditions that adversely affect or are highly likely to adversely affect the health of a subject due to excess weight or high body fat percentage. In the present invention, the term "obesity" may be used interchangeably with "adipositas" since it has substantially the same sense as it. In particular, obesity is known as a major cause of insulin resistance due to an increase in lipid metabolites (Proc. Nutr. Soc. 63: 375-380). Obesity increases the onset of arteriosclerosis, cardiovascular diseases (stroke and ischemic cardiovascular diseases), high blood pressure, diabetes, hyperlipidemia, fatty liver, cholelithiasis, obstructive sleep apnea, menstrual irregularity, polycystic ovary diseases, infertility, decreased libido, depression, degenerative arthritis, gout, and the like. The prophylactic or therapeutic effect for diseases caused by obesity can be expected through the prophylactic or therapeutic effect of the composition of the present invention. In the present invention, the obesity may be simple obesity, symptomatic obesity, childhood obesity, adult obesity, hyperplastic obesity, hypertrophic obesity, upper body obesity, lower body obesity, visceral fat type obesity or subcutaneous fat type obesity.

As used herein, the term "diabetes" refers to a type of metabolic disease that shows symptoms such as insufficient insulin secretion or abnormal function, is characterized by high blood sugar (hyperglycemia) meaning an increase in concentration of blood glucose and exhibits various symptoms and signs due to high blood sugar, resulting in the excretion of glucose in the urine. Preferably, the diabetes may be obese diabetes, but the present invention is not limited thereto.

As used herein, the term "hypertension" is characterized by an increased perfusion blood pressure of blood flowing through an artery. When the systolic blood pressure is 140 mmHg or more and the diastolic blood pressure is 90 mmHg or more, hypertension can usually be diagnosed. Hypertension has no clear symptoms and includes primary (or essential) hypertension for which the exact cause is unknown, and secondary hypertension caused by kidney disease, endocrine disease, and preeclampsia. Most hypertension (90-95%) is primary hypertension and it is presumed that hypertension is caused by environmental factors such as obesity, stress, drinking alcohol, and smoking as well as genetic causes, but is not limited thereto.

As used herein, the term "arteriosclerosis" is defined as a phenomenon in which the elasticity of the artery decreases, fat accumulates on the inner surface of the artery wall, and abnormal tissue proliferates, thereby narrowing the width of the artery wall. Arteriosclerosis is a term that means pathological changes in arteries and the disease name is determined depending on the organ in which the problem is caused by arteriosclerosis, for example, cerebral infarction due to arteriosclerosis, myocardial infarction due to coronary arteriosclerosis, or the like, but is not limited thereto.

As used herein, the term "hyperlipidemia" is a disease caused by a large amount of fat in the blood due to abnormal metabolism of fat such as triglycerides and cholesterol. Specifically, hyperlipidemia refers to a condition in which lipid components such as triglycerides, LDL cholesterol, and free fatty acids in the blood are increased. Hyperlipidemia includes hypercholesterolemia or hypertriglyceridemia, but is not limited thereto.

As used herein, the term "fatty liver" refers to a state in which fat is accumulated in hepatocytes in an excessive amount due to a disorder of fat metabolism in the liver and is defined as a case in which the weight of fat in the liver is 5% or more, causing various diseases such as angina pectoris, myocardial infarction, stroke, arteriosclerosis, fatty liver and pancreatitis. Fatty liver is divided into alcohol-induced alcoholic fatty liver and non-alcoholic fatty liver disease (NAFLD), but is not limited thereto.

As used herein, the term "nonalcoholic fatty liver disease" refers to a fatty liver disease, the cause of which is not alcohol, and includes a series of processes ranging from simple steatosis to non-alcoholic steatohepatitis (NASH) and hepatic cirrhosis. The causes of nonalcoholic fatty liver disease include side effects of antiarrhythmic drugs, antiviral drugs, steroids, cytotoxic drugs, and the like, excessive calorie intake through carbohydrates, obesity, diabetes, and some genetic causes, but are not limited thereto.

As used herein, the term "hyperinsulinemia" is a disease characterized by presence of excessive insulin in the blood. Insulin is a hormone that regulates the level of blood sugar secreted by the pancreas and promotes the influx of sugar into muscles and other peripheral tissues. Therefore, hyperinsulinemia may occur due to a disorder of the pancreas, which is an insulin-secreting organ, and the greatest cause of the onset is insulin resistance, but is not limited thereto.

As used herein, the term "insulin resistance" refers to a condition in which cells cannot effectively burn glucose because they do not respond to insulin that lowers blood sugar. When insulin resistance is high, the human body recognizes that it needs insulin and produces more insulin. This causes hyperinsulinemia, hypertension or dyslipidemia, as well as heart disease and diabetes, but is not limited thereto.

As used herein, the term "insulin resistance syndrome" is a generic term for diseases caused by the insulin resistance. Insulin resistance syndrome is characterized by cellular resistance to insulin action, hyperinsulinemia, an increase in very low density lipoprotein (VLDL) and triglycerides, a decrease in high density lipoprotein (HDL), and hypertension and is recognized as a risk factor for disease and type 2 diabetes.

As used herein, the term "meta-inflammation" is a chronic and low-grade inflammation and means an inflammatory response that occurs due to an excessive supply of nutrients or metabolites. In particular, the chronic inflammatory response caused by obesity is known to play an important role in the process of increased insulin resistance and metabolic abnormalities.

In the present invention, the composition may be a pharmaceutical composition or a food composition.

In the present invention, unless otherwise specified, the pharmaceutical composition or food composition may be the same as the content described with regard to the composition for suppressing weight gain.

As used herein, the term "prevention" refers to any action that suppress or delays the onset of obesity or obesity-related diseases by administration of the composition according to the present invention.

As used herein, the term "treatment" means any action that can ameliorate or beneficially alter the symptoms of obesity or obesity-related diseases by administration of the composition according to the present invention.

In another aspect, the present invention is related to the use of a *Lactobacillus fermentum* strain for administration in combination with regulatory T cells for the amelioration, prevention or treatment of metabolic diseases.

In another aspect, the present invention is related to the use of a *Lactobacillus fermentum* strain and regulatory T cells for the preparation of a composition for ameliorating, preventing or treating metabolic diseases.

In another aspect, the present invention is related to a method of ameliorating, preventing or treating metabolic diseases including administering a *Lactobacillus fermentum* strain in combination with regulatory T cells to a subject.

In the present invention, the *Lactobacillus fermentum* strain preferably includes at least one selected from the group consisting of a *Lactobacillus fermentum* GB-102 strain deposited with the accession number of KCTC 14105BP and a *Lactobacillus fermentum* GB-103 strain deposited with the accession number of KCTC 14106BP.

In the present invention, the administration and co-administration may have the same features as those disclosed in terms of the composition for suppressing weight gain and method for suppressing weight gain according to the present invention, unless otherwise specified.

### [Deposition Information]

Name of depositary institution: Korea Research Institute of Bioscience and Biotechnology
Accession number: KCTC14105BP
Deposit date: 20200114
Name of depositary institution: Korea Research Institute of Bioscience and Biotechnology
Accession number: KCTC14106BP
Deposit date: 20200114

### Example

Hereinafter, the present invention will be described in more detail with reference to the following examples. However, it will be obvious to those skilled in the art that the following examples are provided only for illustration of the present invention and should not be construed as limiting the scope of the present invention.

### Example 1: Preparation of regulatory T cells (Treg cells)

### Example 1-1. Preparation of lymph node and spleen

In order to obtain mouse-derived regulatory T cells (Treg cells), lymph node and spleen were extracted from B6.Cg-Foxp3tm2Tch/J mice (The Jackson laboratory; distributor: Saeron Bio), transferred to a 50 mL conical tube containing 5 mL of PBS and then stored on ice. 5 mL of FACS buffer shown in Table 1 below was preliminarily charged in a fresh 50 mL conical tube, and 70 um strainers were stacked to prepare lymph node and spleen samples.

Each of the lymph node and spleen was placed on the 70 um strainer and was ground, and the FACS buffer A was allowed to flow over the tissue to obtain cells. The obtained cells were centrifuged at 4°C and 1,300 rpm for 10 minutes, the supernatant was removed, 3 mL of ACK lysis buffer (Lonza, # BP10-548E) was mixed with the cell pellet, and the resulting mixture was allowed to stand at room temperature for 5 minutes. 47 mL of FACS buffer A was added to the resulting product, followed by centrifugation at 1,300 rpm and 4°C for 10 minutes. Then, the supernatant was removed, the residue was lysed with 1 mL of FACS buffer A and the cells were counted.

### Example 1-2: Mouse Treg cell isolation and culture

Only regulatory T cells (Treg cells) from the bone marrow cells obtained in Example 1-1 were isolated using a CD4+ T cell isolation kit, mouse (Miltenyi biotec, #130-115-818) as follows, and then centrifuged at 300×g and 4°C and the supernatant was removed.

MACS buffer (40 µL per 10⁷ cells) was added to the cells to lyse a cell pellet and a biotin-antibody cocktail (10 µL per 10⁷ cells) was added thereto. The cells were incubated at 4°C for 5 minutes, MACS buffer (30 µL/10⁷ cells) was added thereto, and anti-biotin microbeads (20 µL/10⁷ cells) were added thereto. After incubation at 4°C for 10 minutes, the cells were allowed to flow into the pre-wetted LS column to obtain the supernatant that passed through the column. The same process as above was repeated, the result was centrifuged at 300×g and at 4°C, and the supernatant was removed.

The cells were lysed in 1 mL of FACS buffer A, added with 20 µL CD4-Pacific Blue (BioLegend, cat# 100531), and the result was allowed to stand on ice for 20 minutes. Then, 4 mL of FACS buffer was further added and centrifuged at 1,300 rpm and 4°C for 5 minutes. The supernatant was removed, 5 mL of FACS buffer was added to the residue and the result was centrifuged at 1,300 rpm and 4°C for 5 minutes. Then, the supernatant was removed and the cells were lysed in 10 mL MACSQuant Tyto Running Buffer (Miltenyii biotec/# 130-107-207). Finally, the CD4+Foxp3GFP+ cells were isolated with MACSQuant^{®} Tyto^{®}.

The isolated regulatory T cells (Treg cells) were lysed in 1 mL of GC-RPMI medium shown in Table 1 and then were counted. The cells were seeded into GC-RPMI medium at 5×10⁵ cells/mL based on the measured number of Treg cells, treated with rmIL-2 at a concentration of 100 nM/mL and cultured for 7 days.

**[Table 1]**

| **For 500 mL** | **Volume** | **Final Concentration** |
|---|---|---|
| FBS | 50 ml | 10% |
| 1M HEPES | 5 ml | 20 mM |
| 10,000 U/ml Penicillin & 10,000 µg/ml Streptomycin | 5 ml | 100 U/ml Penicillin & 100 µg/ml Streptomycin |
| 50 mg/ml Gentamicin | 0.5 ml | 50 µg/ml |
| 100 mM Sodium Pyruvate | 5 ml | 1 mM |
| 55 mM 2-Mercaptoethanol | 0.5 ml | 55 µM |
| 100 mM NEAA | 5 ml | 1 mM |

### Example 2: Isolation and identification of Lactobacillus fermentum strains

### Example 2-1: Isolation of strains

The *Lactobacillus fermentum* strain of the present invention was isolated from a vaginal sample of a healthy woman who visited the hospital for the purpose of medical examination. Specifically, a vaginal sample was collected with a cotton swab, lining-inoculated in Rogosa SL plate medium, and incubated in an anaerobic chamber at 37°C for 48 hours. When colonies of bacteria grew, single colonies were subcultured in fresh Rogosa SL plate medium for pure isolation. After pure isolation, strain culture was performed using MRS medium.

### Example 2-2: Selection of strain having inhibitory activity against fat accumulation

In order to select a strain having inhibitory activity against fat accumulation, the ability to inhibit pancreatic lipase activity and the ability to inhibit the differentiation of 3T3-L1 preadipocytes into adipocytes were confirmed.

Specifically, the strain of Example 2-1 was diluted to a concentration of 0.1 mg/mL, a 0.167 mM p-nitrophenyl palmitate (PNP; Sigma, USA) solution, a 0.061 M Tris-HCl buffer (pH 8.5), and a 0.3 mg/mL lipase solution were placed on a plate and reacted at 25°C for 10 minutes, followed by absorbance measurement at 405 nm, to determine the ability to inhibit pancreatic lipase activity.

In addition, the inhibition of differentiation of 3T3-L1 preadipocytes into adipocytes was measured using Oil Red O (Sigma, USA), which reacts specifically with adipocytes produced in the cells. After fat differentiation, the medium was removed, washed twice with PBS, fixed with 10% formalin at 4°C for 1 hour, washed twice with 60% isopropanol, and stained with 0.5% Oil Red O solution at room temperature for 30 minutes. After staining, the dye solution was removed and washed twice with distilled water. When distilled water was completely evaporated, isopropyl alcohol was added thereto and absorbance was measured at 520 nm.

Finally, 3T3-L1 cell viability in the strain obtained in Example 2-1 was measured using the MTT (3-(4,5-dimethylthiazol-2-yl)-2,5-diphenyltetrazolium bromide) method. The 3T3-L1 cells were seeded into a 96-well plate at a concentration of 16×10⁴ cells/well and cultured for 24 hours and the medium was removed. Here, lactic acid bacteria samples (100, 1,000 µg/mL) diluted by concentration were added to 100 µL of fresh DMEM medium and cultured for 24 hours. Then, 20 µL of MTT (Sigma, USA) solution prepared at 5 mg/mL was added to the resulting medium and incubated at 37°C for 4 hours. After incubation, the supernatant was removed, 200 µL of dimethyl sulfoxide (DMSO) was added to the residue, and absorbance was measured at 546 nm.

Based on the results above, fat accumulation inhibitory effect of each strain was confirmed. Among the prepared strains, the *Lactobacillus fermentum* GB102 and GB103 strains, which showed the highest fat cell accumulation inhibitory effect and the lowest cytotoxicity rate, were finally selected.

### Example 2-3. Molecular biological identification of selected strain

For the identification of the finally selected *Lactobacillus fermentum* GB-102 and GB-103 strains, 16S rRNA gene sequence was analyzed. The sequences obtained through the Sanger sequencing method using 27F and 1492R primers targeting the 16S rRNA gene of bacteria are shown in Table 2 below.

**[Table 2]**

| **Name** | **Sequence (5' to 3')** | **SEQ NO.** |
|---|---|---|
| GB102_16S_fu ll | GCAAGTCGAACGCGTTGGCCCAATTGATTGATGGTGCTTGCACC TGATTGATTTTGGTCGCCAACGAGTGGCGGACGGGTGAGTAACA CGTAGGTAACCTGCCCAGAAGCGGGGGACAACATTTGGAAACAG ATGCTAATACCGCATAACAACGTTGTTCGCATGAACAACGCTTA AAAGATGGCTTCTCGCTATCACTTCTGGATGGACCTGCGGTGCA TTAGCTTGTTGGTGGGGTAATGGCCTACCAAGGCGATGATGCAT AGCCGAGTTGAGAGACTGATCGGCCACAATGGGACTGAGACACG GCCCATACTCCTACGGGAGGCAGCAGTAGGGAATCTTCCACAAT GGGCGCAAGCCTGATGGAGCAACACCGCGTGAGTGAAGAAGGGT TTCGGCTCGTAAAGCTCTGTTGTTAAAGAAGAACACGTATGAGA GTAACTGTTCATACGTTGACGGTATTTAACCAGAAAGTCACGGC TAACTACGTGCCAGCAGCCGCGGTAATACGTAGGTGGCAAGCGT TATCCGGATTTATTGGGCGTAAAGAGAGTGCAGGCGGTTTTCTA AGTCTGATGTGAAAGCCTTCGGCTTAACCGGAGAAGTGCATCGG AAACTGGATAACTTGAGTGCAGAAGAGGGTAGTGGAACTCCATG TGTAGCGGTGGAATGCGTAGATATATGGAAGAACACCAGTGGCG AAGGCGGCTACCTGGTCTGCAACTGACGCTGAGACTCGAAAGCA TGGGTAGCGAACAGGATTAGATACCCTGGTAGTCCATGCCGTAA ACGATGAGTGCTAGGTGTTGGAGGGTTTCCGCCCTTCAGTGCCG GAGCTAACGCATTAAGCACTCCGCCTGGGGAGTACGACCGCAAG GTTGAAACTCAAAGGAATTGACGGGGGCCCGCACAAGCGGTGGA GCATGTGGTTTAATTCGAAGCTACGCGAAGAACCTTACCAGGTC TTGACATCTTGCGCCAACCCTAGAGATAGGGCGTTTCCTTCGGG AACGCAATGACAGGTGGTGCATGGTCGTCGTCAGCTCGTGTCGT GAGATGTTGGGTTAAGTCCCGCAACGAGCGCAACCCTTGTTACT AGTTGCCAGCATTAAGTTGGGCACTCTAGTGAGACTGCCGGTGA CAAACCGGAGGAAGGTGGGGACGACGTCAGATCATCATGCCCCT TATGACCTGGGCTACACACGTGCTACAATGGACGGTACAACGAG TCGCGAACTCGCGAGGGCAAGCAAATCTCTTAAAACCGTTCTCA GTTCGGACTGCAGGCTGCAACTCGCCTGCACGAAGTCGGAATCG CTAGTAATCGCGGATCAGCATGCCGCGGTGAATACGTTCCCGGG CCTTGTACACACCGCCCGTCACACCATGAGAGTTTGTAACACCC AAAGTCGGTGGGGTAACCTTTTAGGAGCCAGCC | 1 |
| GB103_16S_fu ll | ATACATGCAAGTCGAACGCGTTGGCCCAATTGATTGATGGTGCT TGCACCTGATTGATTTTGGTCGCCAACGAGTGGCGGACGGGTGA GTAACACGTAGGTAACCTGCCCAGAAGCGGGGGACAACATTTGG AAACAGATGCTAATACCGCATAACAGCGTTGTTCGCATGAACAA CGCTTAAAAGATGGCTTCTCGCTATCACTTCTGGATGGACCTGC | 2 |
| | GGTGCATTAGCTTGTTGGTGGGGTAACGGCCTACCAAGGCGATG ATGCATAGCCGAGTTGAGAGACTGATCGGCCACAATGGGACTGA GACACGGCCCATACTCCTACGGGAGGCAGCAGTAGGGAATCTTC CACAATGGGCGCAAGCCTGATGGAGCAACACCGCGTGAGTGAAG AAGGGTTTCGGCTCGTAAAGCTCTGTTGTTAAAGAAGAACACGT ATGAGAGTAACTGTTCATACGTTGACGGTATTTAACCAGAAAGT CACGGCTAACTACGTGCCAGCAGCCGCGGTAATACGTAGGTGGC AAGCGTTATCCGGATTTATTGGGCGTAAAGAGAGTGCAGGCGGT TTTCTAAGTCTGATGTGAAAGCCTTCGGCTTAACCGGAGAAGTG CATCGGAAACTGGATAACTTGAGTGCAGAAGAGGGTAGTGGAAC TCCATGTGTAGCGGTGGAATGCGTAGATATATGGAAGAACACCA GTGGCGAAGGCGGCTACCTGGTCTGCAACTGACGCTGAGACTCG AAAGCATGGGTAGCGAACAGGATTAGATACCCTGGTAGTCCATG CCGTAAACGATGAGTGCTAGGTGTTGGAGGGTTTCCGCCCTTCA GTGCCGGAGCTAACGCATTAAGCACTCCGCCTGGGGAGTACGAC CGCAAGGTTGAAACTCAAAGGAATTGACGGGGGCCCGCACAAGC GGTGGAGCATGTGGTTTAATTCGAAGCTACGCGAAGAACCTTAC CAGGTCTTGACATCTTGCGCCAACCCTAGAGATAGGGCGTTTCC TTCGGGAACGCAATGACAGGTGGTGCATGGTCGTCGTCAGCTCG TGTCGTGAGATGTTGGGTTAAGTCCCGCAACGAGCGCAACCCTT GTTACTAGTTGCCAGCATTAAGTTGGGCACTCTAGTGAGACTGC CGGTGACAAACCGGAGGAAGGTGGGGACGACGTCAGATCATCAT GCCCCTTATGACCTGGGCTACACACGTGCTACAATGGACGGTAC AACGAGTCGCGAACTCGCGAGGGCAAGCAAATCTCTTAAAACCG TTCTCAGTTCGGACTGCAGGCTGCAACTCGCCTGCACGAAGTCG GAATCGCTAGTAATCGCGGATCAGCATGCCGCGGTGAATACGTT CCCGGGCCTTGTACACACCGCCCGTCACACCATGAGAGTTTGTA ACACCCAAAGTCGGTGGGGTAACCTTTTAGGAGCCAGCC | |

As a result, as shown in Table 3, the GB102 strain showed the highest sequence similarity with the standard strain (type strain) of *Lactobacillus fermentum* (*Limosilactobacillus fermentum*) and the next highest with *Lactobacillus gorillae* (*Limosilactobacillus gorillae*)*.* This indicates that the GB102 and the GB103 strains are strains similar to *Lactobacillus fermentum* and *Lactobacillus gorillae.*

**[Table 3]**

| Strain | Species | Standard strain name | Sequence similarity (%) |
|---|---|---|---|
| GB102 | *L. fermentum* | CECT 562 (T) | 99.79 |
| | *L. gorillae* | KZ01 (T) | 98.26 |
| GB103 | *L. fermentum* | CECT 562 (T) | 99.93 |
| | *L. gorillae* | KZ01 (T) | 98.13 |

### Example 3. Analysis of genome and comparative genome of Lactobacillus fermentum strain

In order to identify and characterize the genome-based species of the GB102 and GB103 strains, using next-generation sequencing technology (NGS) and bioinformatics technology, the strain genome was completely sequenced and the functions of genes contained in the genome were predicted. In addition, the specificity of the strains was confirmed through comparative analysis with the completely decoded genome sequence of the same species. Each of the strain was cultured in an anaerobic chamber at 37°C in MRS broth for 4 hours, and genomic DNA was extracted from the culture using a MG genomic DNA purification kit (MGMED, Inc., Korea). In order to obtain long read data with an average length of 10 kb or more, sequence analysis was performed using PacBio RS II equipment and, in order to produce fragment sequence data having a sequence length of less than 500 bp with high accuracy to compensate for long read data with low accuracy, sequence analysis was performed using a NovaSeq 6000. Long read data were assembled into the high-quality draft GB102 genome using the HGAP2 pipeline of the SMRT analysis server. SNP and indel errors that may be present in the assembled genome draft sequence were corrected through long read data and fragment sequence data. From the completed genome sequence, CDSs were predicted using the Prodigal program, and rRNA and tRNA were predicted using RFAM tool. CDS functions were predicted by performing a homology search (using BLAST algorithm) based on the published UniProt database, GenBank NR database, Subsystem database, PFAM database, and COG database. The average nucleotide identity (ANI) was calculated using the Jspecies program from the genome sequence of standard strain B1 28^{T} (= ATCC 14931^{T}) of *Lactobacillus fermentum* species published on GenBank, *Lactobacillus gorillae* standard strain KZ01^{T}, *Lactobacillus gastricus* standard strain DSM 16045^{T} and the GB102 and GB103 strains. The result of the analysis showed that the GB102 and GB103 strains had an ANI value of 95% or more with the *Lactobacillus fermentum* standard strain, which demonstrates that the GB102 and GB103 strains belong to the *Lactobacillus fermentum* species (Table 4). In addition, the GB102 and GB103 strains are not 100% identical to the standard strain, which indicates that the GB102 and GB103 strain is a novel species that has not been isolated and reported previously. In addition, the GB102 and GB103 strains were also identified as different strains due to the long genomic distance thereof. The present inventors named the GB102 and GB103 bacteria "*Lactobacillus fermentum* GB102" (Accession No.: KCTC 14105BP) and "*Lactobacillus fermentum* GB103" (Accession No.: KCTC 14106BP), and deposited the same in the Korea Cell Line Bank (Korean collection for type cultures, KCTC) located at the Korea Research Institute of Bioscience and Biotechnology on January 14, 2020.

**[Table 4]**

| Strain name | Genome size (bp) | GC ratio | ANI values | | | | |
|---|---|---|---|---|---|---|---|
| | | | GB102 | GB103 | B1 28 | KZ01 | DSM 16045 |
| GB102 | 2,039,432 | 51.88 | --- | **98.43** | **99.14** | 79.31 | 68.77 |
| GB103 | 2,260,513 | 51.25 | **98.51** | --- | **98.42** | 79.72 | 69.23 |
| B1 28 | 1,905,587 | 52.30 | **99.09** | **98.51** | --- | 79.42 | 68.66 |
| KZ01 | 1,641,621 | 48.11 | 79.05 | 79.37 | 79.11 | --- | 68.07 |
| DSM 16045 | 1,848,461 | 41.64 | 68.22 | 68.38 | 68.02 | 68.18 | --- |

### Example 4. Genome-based phylogenetic analysis to determine evolutionary location of each strain among Lactobacillus fermentum species

Genome-based phylogenetic analysis was performed to confirm the evolutionary position of each strain among *Lactobacillus fermentum* species. The fully decoded genome sequences of 34 *Lactobacillus fermentum* strains published on GenBank were compared with the GB102 and GB103 genome sequences. The fully decoded genome of *Lactobacillus gastricus* LG045 strain was used as an outgroup to draw a genome-based phylogenetic tree. Ortholog clustering of protein-coding genes present in the genomes of strains was performed using the orthoMCL program. 930 genes that are present in the genome of all 37 strains and exist singly in the genome of each strain were selected and a phylogenetic tree was created using the RAxML program. The created phylogenetic tree indicates that the GB102 and GB103 strains are strains derived from a lineage different from the standard strain (B1 28^{T}) or the existing patent strain, the NCC2970 strain from Nestlé Culture Collection (NCC) (as shown in FIG. 1) .

### Example 5: Confirmation of effects of co-administration of Treg cells and Lactobacillus fermentum strain on obesity and metabolic diseases

### Example 5-1. Construction and test method of all experimental groups of obese mice induced by high-fat diet

B6 mice (C57BL/6, male, Coretech Korea co., Ltd.) were used as animal test models.

A therapeutic effect was tested in therapeutics model, established by administering lactobacillus, which is expected to have an immune enhancing effect due to changes in the intestinal microflora of mice that were fed a 60% kcal high fat diet to induce high obesity, and Treg cells, which are related to obesity symptoms and blood sugar control, singly or in combination with GB-102 or GB-103. In order to establish the therapeutic model, 140 4-week-old C57BL/6 male mice were obtained and were acclimatized for 2 weeks, obesity was induced with a 60% kcal high-fat diet for 8 weeks, and the body weight was measured once a week, a total of 8 times during 7 weeks of the test.

As shown in the table below, each group was set and the body weight measurement and glucose tolerance test (IPGTT) were performed on the experimental animals to confirm the therapeutic efficacy for obesity and metabolic diseases.

Specifically, in order to confirm the effect of Treg cells and *Lactobacillus fermentum* GB-102 or GB-103 alone or in combination as a therapeutic agent for obesity in mice with obesity induced by a high-fat diet, the mice were classified into a total of seven experimental groups (Table 5). At this time, *Lactobacillus fermentum* GB-102 or GB-103 was prepared as a lyophilized live cell powder, diluted in PBS, and orally administered once daily at a dose of 5 × 10⁹ CFU/head/200 µl. The Treg cells cultured for 7 days in Example 1-2 were collected in a 50 mL conical tube, and centrifuged at 1,300 rpm and 4°C for 10 minutes, the supernatant was removed, the result was diluted with PBS and intravenous administration was performed once a week at a dose of 1 × 10⁶ cells/head/200 µl. Additionally, the response of the mice to administration, the gloss of the fur, and abnormal reactions of animals upon autopsy were observed and stress factors such as observation and group caging were minimized.

**[Table 5]**

| Item | Test group | | | Dose | Administration method |
|---|---|---|---|---|---|
| G1 | Control (NCD) | | | PBS + PBS | - |
| G2 | Control (HFD) | | | PBS + PBS | - |
| G4 | Treg cells alone | | | PBS + 1 × 10⁶ cell/head/200µl | Intravenous administration, once a week for 7 weeks (total 7 times) |
| G5 | GB-102 | | | PBS + 5 × 10⁹ cfu/head/200µl | Oral administration, once a day for 7 weeks (total 49 times) |
| G7 | Treg cells + GB-102 | | Treg | PBS + 1 × 10⁶ cell/head/200µl | Intravenous administration, once a week for 7 weeks (total 7 times) |
| | | | GB-102 | PBS + 5 × 10⁹ cfu/head/200 µl | Oral administration, once a day for 7 weeks (total 49 times) |
| G8 | GB-103 | | | PBS + 5 × 10⁹ cfu/head/200µl | Oral administration, once a day for 7 weeks (total 49 times) |
| G9 | Treg cells + GB-103 | Treg | | PBS + 1 × 10⁶ cell/head/200µl | Intravenous administration, once a week for 7 weeks (total 7 times) |
| | | GB-103 | | PBS + 5 × 10⁹ cfu/head/200µl | Oral administration, once a day for 7 weeks (total 49 times) |

### Example 5-2: Effects of single or co-administration of Treg cell and Lactobacillus fermentum GB102 and GB103 on weight change

As can be seen from the result of measurement of the change in body weight, the group administered the Treg cells alone and the group administered the *Lactobacillus fermentum* GB-102 alone did not greatly reduce body weight, whereas the group administered a combination of Treg cells and *Lactobacillus fermentum* GB-102 exhibited a remarkable weight loss effect (Table 6 and FIG. 2).

**[Table 6]**

| **Gain (AVG)** | **0 day** | **1 W** | **2 W** | **3 W** | **4 W** | **5 W** | **6 W** | **7 W** |
|---|---|---|---|---|---|---|---|---|
| G1 | 0.00 | -0.38 | -0.75 | -0.31 | 0.44 | 1.04 | 1.30 | 1.65 |
| | 0.00 | ±0.28 | ±0.45 | ±0.61 | ±0.73 | ±0.88 | ±0.83 | ±0.75 |
| G2 | 0.00 | 1.63 | 3.06 | 4.55 | 5.97 | 6.99 | 7.63 | 8.46 |
| | 0.00 | ±0.31 | ±0.25 | ±0.30 | ±0.36 | ±0.41 | ±0.51 | ±0.67 |
| G4 | 0.00 | 0.43 | 1.31 | 2.51 | 3.17 | 4.13 | 4.82 | 5.86 |
| | 0.00 | ±0.31 | ±0.25 | ±0.30 | ±0.36 | ±0.41 | ±0.51 | ±0.67 |
| G5 | 0.00 | 0.99 | 1.74 | 3.07 | 3.10 | 4.31 | 5.48 | 6.53 |
| | 0.00 | ±0.18 | ±0.29 | ±0.42 | ±0.69 | ±0.74 | ±0.70 | ±0.77 |
| G7 | 0.00 | 0.42 | 1.95 | 3.13 | 2.56 | 3.58 | 4.36 | 4.74 |
| | 0.00 | ±0.17 | ±0.23 | ±0.28 | ±0.64 | ±0.44 | ±0.40 | ±0.41 |

| | | | | | | | | |
|---|---|---|---|---|---|---|---|---|
| Data are expressed as Mean±SEM G1: Negative control group(NCD), G2: Negative cont group(HFD), G4: Treg cells alone, G5: GB-102, G7: Treg+GB-102 | | | | | | | | |

In addition, it can be seen that, the group administered Treg cells alone had no weight loss effect, whereas the group administered the *Lactobacillus fermentum* GB-103 alone exhibited a weight loss effect compared to a control group with a high-fat diet. In addition, it can be seen that the group administered a combination of the Treg cells and *Lactobacillus fermentum* GB-103 exhibited a remarkable weight loss effect compared to the group administered each test substance alone (Table 7 and FIG. 3).

**[Table 7]**

| **Gain (AVG)** | **0 day** | **1 W** | **2 W** | **3 W** | **4 W** | **5 W** | **6 W** | **7 W** |
|---|---|---|---|---|---|---|---|---|
| G1 | 0.00 | -0.38 | -0.75 | -0.31 | 0.44 | 1.04 | 1.30 | 1.65 |
| | 0.00 | ±0.28 | ±0.45 | ±0.61 | ±0.73 | ±0.88 | ±0.83 | ±0.75 |
| G2 | 0.00 | 1.63 | 3.06 | 4.55 | 5.97 | 6.99 | 7.63 | 8.46 |
| | 0.00 | ±0.31 | ±0.25 | ±0.30 | ±0.36 | ±0.41 | ±0.51 | ±0.67 |
| G4 | 0.00 | 0.43 | 1.31 | 2.51 | 3.17 | 4.13 | 4.82 | 5.86 |
| | 0.00 | ±0.31 | ±0.25 | ±0.30 | ±0.36 | ±0.41 | ±0.51 | ±0.67 |
| G8 | 0.00 | 0.45 | 1.19 | 1.90 | 2.23 | 3.50 | 4.22 | 4.83 |
| | 0.00 | ±0.20 | ±0.43 | ±0.53 | ±0.59 | ±0.60 | ±0.59 | ±0.64 |
| G9 | 0.00 | -0.25 | 0.84 | 1.59 | 0.96 | 2.66 | 3.26 | 3.61 |
| | 0.00 | ±0.08 | ±0.09 | ±0.31 | ±0.45 | ±0.51 | ±0.55 | ±0.35 |

| | | | | | | | | |
|---|---|---|---|---|---|---|---|---|
| Data are expressed as Mean±SEM G1: Negative control group(NCD), G2: Negative control group(HFD), G4: Treg cells alone, G8: GB-103, G9: Treg+GB-103 | | | | | | | | |

### Example 5-3. Intraperitoneal glucose tolerance test (IPGTT) upon co-administration of immune cells and Lactobacillus fermentum strain

Before performing the intraperitoneal glucose tolerance (IPGTT) test, all measured animals were fasted for at least 12 hours. The tail end of the fasted mice was cut and the fasting blood glucose was measured with a blood glucose meter. Glucose was diluted in distilled water to obtain 10% glucose, which was administered in an amount (µl) corresponding to 10 times the body weight of each individual animal. After administration, blood was collected from the tail end at 30-minute intervals for 2 hours and blood glucose was measured with a glucometer.

The change in glucose tolerance (IPGTT) when Treg cells and *Lactobacillus fermentum* GB-102 were administered alone or in combination was observed. The result showed that the group administered the Treg cells alone and the group administered the *Lactobacillus fermentum* GB-102 alone exhibited a blood glucose control effect, whereas the group administered a combination of the Treg cells and *Lactobacillus fermentum* GB-102 reached a low blood glucose peak and the fasting blood glucose level fastest, which indicates that the group administered the combination exhibited excellent blood glucose control effect compared to the group administered alone (Table 8 and FIG. 4).

**[Table 8]**

| **AVG (mg/dl)** | **0 min** | **30 min** | **60 min** | **90 min** | **120 min** |
|---|---|---|---|---|---|
| G1 | 125.90 | 257.20 | 191.60 | 184.10 | 165.10 |
| | ±3.64 | ±7.14 | ±6.71 | ±7.32 | ±9.34 |
| G2 | 157.53 | 350.80 | 232.20 | 220.33 | 202.40 |
| | ±4.48 | ±14.52 | ±12.46 | ±15.32 | ±14.08 |
| G4 | 152.89 | 294.67 | 226.00 | 205.33 | 167.44 |
| | ±6.48 | ±16.09 | ±6.47 | ±5.27 | ±5.96 |
| G5 | 167.40 | 319.30 | 245.60 | 223.90 | 184.70 |
| | ±5.18 | ±11.68 | ±12.35 | ±13.38 | ±9.92 |
| G7 | 159.38 | 255.38 | 196.63 | 192.63 | 169.63 |
| | ±4.99 | ±10.86 | ±16.3 | ±10.04 | ±9.97 |

| | | | | | |
|---|---|---|---|---|---|
| Data are expressed as Mean±SEM G1: Negative control group(NCD), G2: Negative control group(HFD), G4: Treg cells alone, G5: GB-102, G7: Treg+GB-102 | | | | | |

In addition, the change in glucose tolerance (IPGTT) when Treg cells and *Lactobacillus fermentum* GB-103 were administered alone or in combination was observed. The result showed that the group administered the Treg cells alone and the group administered the *Lactobacillus fermentum* GB-103 alone exhibited a blood glucose control effect, whereas the group administered a combination of the Treg cells and *Lactobacillus fermentum* GB-103 reached a low blood glucose peak and the fasting blood glucose level fastest, which indicates that the group administered the combination exhibited an excellent blood glucose control effect compared to the group administered alone (Table 9 and FIG. 5).

**[Table 9]**

| **AVG** | **0 min** | **30 min** | **60 min** | **90 min** | **120 min** |
|---|---|---|---|---|---|
| G1 | 125.90 | 257.20 | 191.60 | 184.10 | 165.10 |
| | ±3.64 | ±7.14 | ±6.71 | ±7.32 | ±9.34 |
| G2 | 157.53 | 350.80 | 232.20 | 220.33 | 202.40 |
| | ±4.48 | ±14.52 | ±12.46 | ±15.32 | ±14.08 |
| G4 | 152.89 | 294.67 | 226.00 | 205.33 | 167.44 |
| | ±6.48 | ±16.09 | ±6.47 | ±5.27 | ±5.96 |
| G8 | 169.78 | 281.11 | 230.67 | 229.22 | 197.67 |
| | ±3.43 | ±13.09 | ±16.12 | ±17.13 | ±12.76 |
| G9 | 138.11 | 238.00 | 186.33 | 172.78 | 162.78 |
| | ±5.94 | ±5.35 | ±7.91 | ±9.76 | ±6.53 |

| | | | | | |
|---|---|---|---|---|---|
| Data are expressed as Mean±SEM G1: Negative control group(NCD), G2: Negative control group (HFD), G4: Treg cells alone, G8: GB-103, G9: Treg+GB-103 | | | | | |

### Industrial Applicability

The composition containing the *Lactobacillus fermentum* strain and regulatory T cells of the present invention and combination therapy using the same exhibit a great weight gain suppression effect. Therefore, the composition containing the *Lactobacillus fermentum* strain and regulatory T cells of the present invention and combination therapy using the same can be easily used to control the body weight of a subject, and are useful in the fields of beauty, livestock, medicine, and the like, in particular, are useful in the fields of food additives, health functional foods, and therapeutic agents for ameliorating, preventing and treating metabolic diseases.

Although specific configurations of the present invention have been described in detail, those skilled in the art will appreciate that this detailed description is provided as preferred embodiments for illustrative purposes and should not be construed as limiting the scope of the present invention. Therefore, the substantial scope of the present invention is defined by the accompanying filed claims and equivalents thereto.

## Claims

1. A composition for suppressing weight gain comprising a *Lactobacillus fermentum* strain as an active ingredient, wherein the composition is administered in combination with regulatory T cells.

2. The composition according to claim 1, wherein the *Lactobacillus fermentum* strain comprises at least one selected from the group consisting of a *Lactobacillus fermentum* GB-102 strain deposited with the accession number of KCTC 14105BP and a *Lactobacillus fermentum* GB-103 strain deposited with the accession number of KCTC 14106BP.

3. The composition according to claim 1, wherein the composition is a pharmaceutical composition or food composition.

4. A composition for preventing or treating a metabolic disease containing a *Lactobacillus fermentum* strain as an active ingredient, wherein the composition is administered in combination with regulatory T cells.

5. The composition according to claim 4, wherein the *Lactobacillus fermentum* strain comprises at least one selected from the group consisting of a *Lactobacillus fermentum* GB-102 strain deposited with the accession number of KCTC 14105BP and a *Lactobacillus fermentum* GB-103 strain deposited with the accession number of KCTC 14106BP.

6. The composition according to claim 4, wherein the metabolic disease is selected from the group consisting of obesity, diabetes, hypertension, arteriosclerosis, hyperlipidemia, hyperinsulinemia, insulin resistance, metabolic inflammation, fasting blood glucose disorder, impaired glucose tolerance and glucose tolerance syndrome.

7. The composition according to claim 4, wherein the composition is a pharmaceutical composition or food composition.
